# EUROPEAN PATENT APPLICATION

(11) **EP 3 189 815 A1**
(43) Date of publication of application: **12.07.2017**
(21) Application number: 16275006.1
(22) Date of filing: 06.01.2016
(51) Int. Cl.: A61F 2/58, B25J 15/08, A61F 4/00

(54) **GRIPPING DEVICE**

(71) Applicant: BAE Systems PLC, London SW1Y 5AD (GB)
(72) Inventor: The designation of the inventor has not yet been filed
(74) Representative: BAE SYSTEMS plc Group IP Department

(57) **Abstract**

A gripping device comprising: a first portion (104) having a first end and a second end and comprising a first arm mount (114) for fitting to a user's forearm; a jaw portion (102) comprising a pair of moveable jaws (108) and coupled to the first end of the first portion (104); a second portion (106) coupled to the second end of the first portion (104) and moveable with respect to the first portion (104) and comprising a second arm mount (124) for fitting to a user's upper arm; and a pull member (126) coupled between the jaw portion (102) and the second body portion (106). Movement of the second portion (106) with respect to the first portion (104) moves the pull member (126) causing it to act on the jaw portion (102) to move the jaws (108) from an open position to a clamped position.

## Description

### FIELD OF THE INVENTION

The present invention relates to gripping devices.

### BACKGROUND

Manual gripping or grabbing tools may be used by people in their daily activities to help with reaching and gripping items.

Typically these tools require the user to have relatively good gripping or squeezing strength to use.

Many users have physical limitations, such as arthritis, a disability, poor hand strength and/or poor dexterity. Manual gripping or grabbing tools tend to be difficult or sometimes impossible for such persons with limitations to operate.

### SUMMARY OF THE INVENTION

In a first aspect, the present invention provides a gripping device comprising: an elongate first body portion having a first end and a second end opposite to the first end, the first body portion comprising a first arm mount for fitting to a forearm of a user; a jaw portion comprising a pair of jaws moveable relative to each other between a fully open position and a fully clamped position thereof, the jaw portion being coupled to the first end of the first body portion; a second body portion coupled to the second end of the first body portion, and moveable with respect to the first body portion, the second body portion comprising a second arm mount for fitting to an upper arm of a user; and a pull member operatively coupled between the jaw portion and the second body portion, the pull member being at least partially housed within the first body portion. Movement of the second body portion with respect to the first body portion is operative to move the pull member thereby to cause the pull member to act on the jaw portion so as to cause the jaws to move from at or proximate to the fully open position thereof towards the fully clamped position thereof.

The jaw portion may further comprise a ratchet system configured to oppose movement of the jaws from at or proximate to the fully clamped position thereof towards the fully open position thereof. The jaw portion may further comprise a release mechanism for releasing the ratchet system so as to allow movement of the jaws from at or proximate to the fully clamped position thereof towards the fully open position thereof. The jaw portion may further comprise a first spring mechanism for moving the jaws from at or proximate to the fully clamped position thereof towards the fully open position thereof responsive to the release mechanism releasing the ratchet system.

The first body portion may be selectively extendible such that a distance between the jaw portion and the second body portion can be varied. The first body portion may further comprise a first elongate member and a second elongate member slidably mounted to the first elongate member. The jaw portion may be coupled to the second elongate member. The second body portion may be coupled to the first elongate member. The first body portion may further comprise a handle portion moveable between a first position and a second position thereof. The first position of the handle portion may be such that the handle portion is engaged with the first elongate member and the second elongate member thereby to fix the relative positions of the first elongate member and the second elongate member. The second position of the handle portion may be such that the handle portion is not engaged with at least one of the first elongate member and the second elongate member thereby allowing the relative movement of the first elongate member and the second elongate member. The first body portion may further comprise a second spring mechanism for moving the handle portion from the second position thereof to the first position thereof.

When the first arm mount is fitted to the forearm of a user and the second arm mount is fitted the upper arm of that user, the movement of the second body portion with respect to the first body portion that moves the pull member and thereby causes the pull member to act on the jaw portion so as to cause the jaws to move from at or proximate to the fully open position thereof towards the fully clamped position thereof may correspond to the user's arm being straightened.

Further movement of the second body portion with respect to the first body portion may be operative to move the pull member and thereby causes the pull member to act on the jaw portion so as to cause the jaws to move from at or proximate to the fully clamped position thereof towards the fully open position thereof. The further movement may be in an opposite direction to that of the movement of the second body portion with respect to the first body portion that moves the pull member and thereby causes the pull member to act on the jaw portion so as to cause the jaws to move from at or proximate to the fully open position thereof towards the fully clamped position thereof. When the first arm mount is fitted to the forearm of a user and the second arm mount is fitted the upper arm of that user, the further movement of the second body portion with respect to the first body portion may correspond to the user's arm being bent at an elbow of the user.

The pull member may be a cable.

At least part of the pull member may be housed in a sleeve.

The second body portion may comprise a roller chain system that, when the first arm mount is fitted to the forearm of a user and the second arm mount is fitted the upper arm of that user, is located at an elbow of that user.

In a second aspect, the present invention provides a method of providing a gripping device, the method comprising: providing an elongate first body portion having a first end and a second end opposite to the first end, the first body portion comprising a first arm mount for fitting to a forearm of a user; coupling a jaw portion the first end of the first body portion, the jaw portion comprising a pair of jaws moveable relative to each other between a fully open position and a fully clamped position thereof; coupling a second body portion to the second end of the first body portion such that the second body portion is moveable with respect to the first body portion, the second body portion comprising a second arm mount for fitting to an upper arm of a user; and coupling a pull member between the jaw portion and the second body portion such that the pull member is at least partially housed within the first body portion and such that movement of the second body portion with respect to the first body portion is operative to move the pull member thereby to cause the pull member to act on the jaw portion so as to cause the jaws to move from at or proximate to the fully open position thereof towards the fully clamped position thereof.

### BRIEF DESCRIPTION OF THE DRAWINGS

Figure 1 is a schematic illustration (not to scale) of an embodiment of a gripping device;
Figure 2 is a schematic illustration (not to scale) showing the gripping device 100 in use;
Figures 3 is a further schematic illustration (not to scale) showing the gripping device in use;
Figure 4 is a schematic illustration (not to scale) showing further details of a jaw portion of the gripping device;
Figure 5 is a schematic illustration (not to scale) showing further details of certain parts of a first body portion of the gripping device; and
Figure 6 is a further schematic illustration (not to scale) showing further details of certain parts of the first body portion.

### DETAILED DESCRIPTION

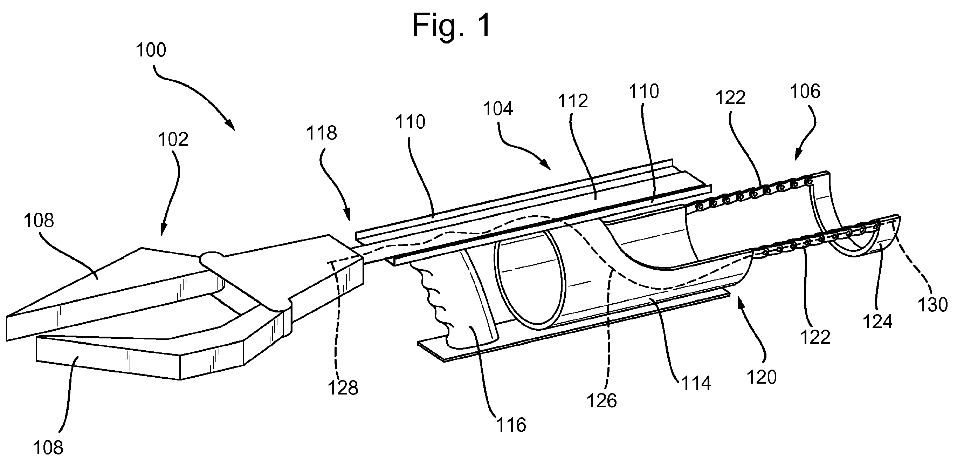

Figure 1 is a schematic illustration (not to scale) of an embodiment of a gripping device 100.

In this embodiment, the gripping device 100 comprises a jaw portion 102, a first body portion 104, and a second body portion 106.

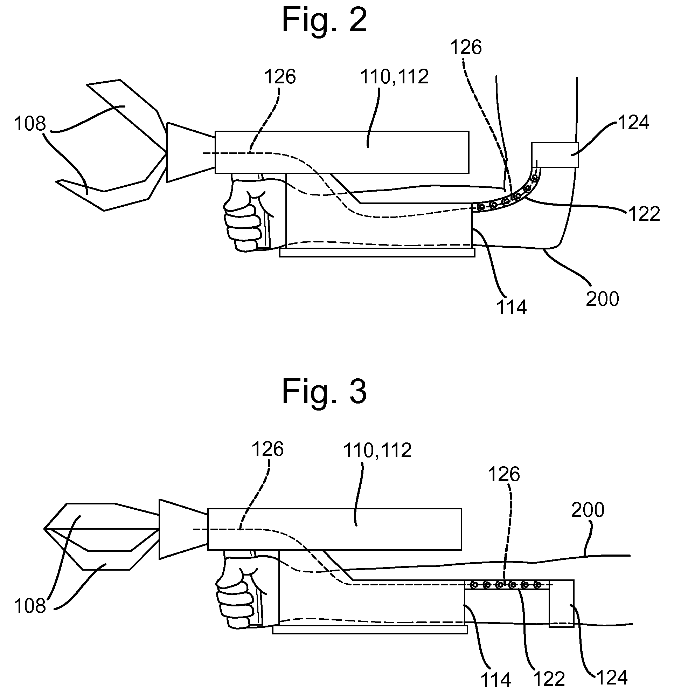

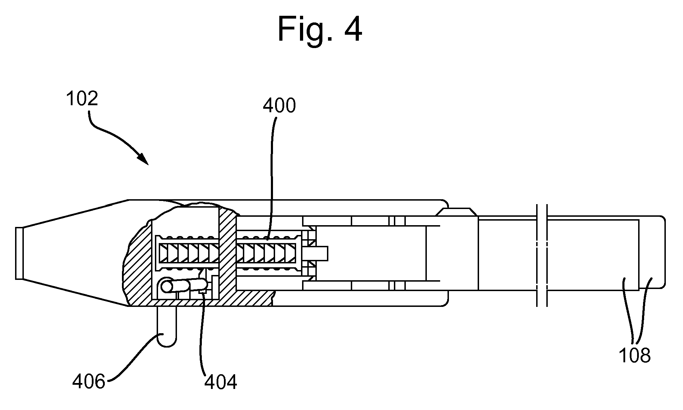

The jaw portion 102 comprises a pair of opposing jaws 108 that are moveable relative to one another. The jaws 108 are moveable between a fully open position and a fully clamped position, and vice versa, as described in more detail later below with reference to Figures 2 and 3. Further details of the jaw portion 102 are described in more detail later below with reference to Figure 4.

The first body portion 104 comprises a first elongate member 110, a second elongate member 112, a first arm mount 114, and a handle 116.

In this embodiment, the first body portion 104 is a substantially elongate structure having a first end 118 and a second end 120 opposite to the first end 118. The jaw portion 102 is attached to the first end 118 of the first body portion 104. The second body portion 106 is attached to the second end 120 of the first body portion 104.

The first elongate member 110 and the second elongate member 112 are mounted together such that they are substantially aligned along their lengths. The first elongate member 110 is slidably mounted to second elongate member 112 such that the second elongate member 112 may slide along a length of the first elongate member 110. In this embodiment, the jaw portion 102 is fixedly attached to a free end of the second elongate member 112 at the first end 118 of the first body portion 104.

The first elongate member 110 is fixedly attached on top of the first arm mount 114.

The first arm mount 114 is shaped so as to fit to a forearm of a user as described in more detail later below with reference to Figures 2 and 3.

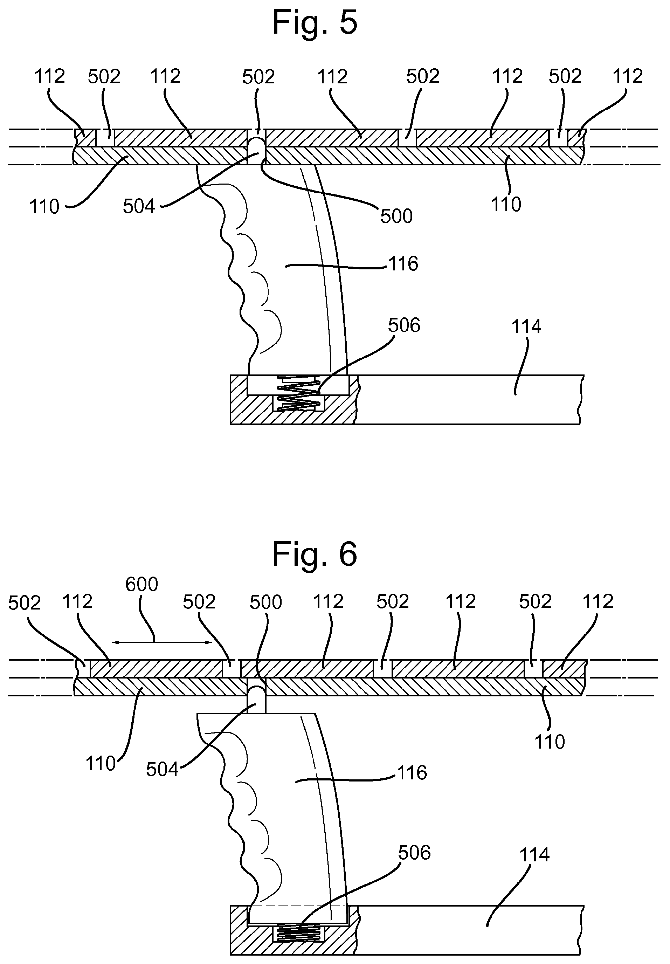

The handle 116 is coupled to the first arm mount 114 at a front end of the first arm mount 114. In particular, in this embodiment, as described in more detail later below with reference to Figures 5 and 6, a lower end of the handle portion 116 is coupled to first arm mount 114, while an upper end of the handle portion 116 selectively engages with the first and second elongate portions 110, 112 to selectively fix the relative positions of the first and second elongate members 110, 112.

The second body portion 106 comprises a hinged chain system 122 and a second arm mount 124. The hinged chain system 122 is disposed between the first arm mount 114 at the second end 120 of the first body portion 104 and the second arm mount 124.

In this embodiment, the hinged chain system 122 comprises two chain structures located at opposite sides of the gripping device 100. Each chain structure of the hinged chain system 122 has the same or a similar structure to that of a roller chain, and comprises a series of connected chain links attached together to form an elongate chain.

The second arm mount 124 is shaped so as to fit to an upper arm of a user as described in more detail later below with reference to Figures 2 and 3.

In this embodiment, the gripping device 100 further comprises a cable 126. The cable 126 coupled together the jaw portion 102 and the second arm mount 124. In particular, the cable 126 is attached at its first end 128 to the jaw portion 102 and at its second end 130, which is opposite to the first end 128, to the second arm mount 124. In this embodiment, the cable 126 runs from the jaw portion 102, through the first body portion 104 from the first end 118 of the first body portion 104 to the second end 120 of the first body portion 104, and then through a chain structure of the hinged chain system 122 to the second arm mount 124. In this embodiment, when passing through the first body portion 104, the cable 126 passes through at least part of the first elongate member 110 and then through at least part of the first arm mount 114.

In some embodiments, at least part of the cable 126 is housed in a sleeve. The cable 126 may be moveable inside the sleeve in a direction along the length of the cable 126. In some embodiments, the sleeve is fixedly attached to some other component of the gripping device 100, for example, the first arm mount 114.

Figures 2 and 3 are a schematic illustration (not to scale) showing the gripping device 100 in use.

In this embodiment, the gripping device 100 is fitted to a user's arm 200 such that the first arm mount 114 is fitted to a forearm of the user, and the second arm mount 124 is fitted to the upper arm of the user. Also, the user grips the handle 116 with their hand.

Preferably, at least one arm mount 114, 124 comprises an adjustable arm straps to provide support to the arm & forearm. The adjustable arm straps may be plastic and coated with a foam material for user comfort. The adjustable arm straps allow different sized arms to be accommodated by the gripping device 100.

In Figure 2, the user's arm 200 is bent at the elbow. In this state, the cable 126 within the gripping device 100 is relatively slack. Also, in this state, the jaws 108 of the jaw portion 102 are in their fully open configuration.

The user straightening his or her arm at the elbow (i.e. moving from the state shown in Figure 2 to that shown in Figure 3), causes the second arm mount 124 to pull the cable 126 in a direction towards the second arm mount 124, i.e. away from the jaw portion 102. Thus, the cable 126 exerts a pulling force on the jaw portion 102, thereby actuating the jaw portion 102 and causing the jaws 108 to move from their fully open configuration (Figure 2) to their fully clamped or closed configuration (Figure 3).

In this way, the user may operate the gripping device 100 to grip an object.

In some embodiments, the user bending his or her arm at the elbow (i.e. moving from the state shown in Figure 3 to that shown in Figure 2), causes the second arm mount 124 to release the tension on the cable 126. Thus, the pulling force exerted on the jaw portion 102 by the cable 126 is reduced or stopped. This may cause the jaws 108 of the jaw portion to move from their fully clamped configuration (Figure 3) back towards their fully open configuration (Figure 2), for example, under action of a sprung return mechanism in the jaw portion 102.

In some embodiments, the cable is sufficiently rigid such that the user bending his or her arm at the elbow (i.e. moving from the state shown in Figure 3 to that shown in Figure 2), causes the second arm mount 124 to push the cable 126 in a direction towards the first arm mount 114, i.e. towards the jaw portion 102. Thus, the cable 126 exerts a pushing force on the jaw portion 102, thereby actuating the jaw portion 102 and causing the jaws 108 to move from their fully clamped configuration (Figure 3) towards their fully open configuration (Figure 2).

In this way, the user may operate the gripping device 100 to release a gripped object.

Figure 4 is a schematic illustration (not to scale) showing further details of the jaw portion 102. Figure 4 shows a top-down view of the jaw portion 102.

In this embodiment, the jaw portion 102 further comprises a ratchet mechanism 400, a return mechanism 404, and a release pin 406.

In this embodiment, the ratchet mechanism 400 is coupled to the jaws 108. The ratchet mechanism 400 is configured to allow the jaws 108 to move towards each other, i.e. in a direction from the fully open configuration of the jaws 108 to the fully closed configuration of the jaws 108. The ratchet mechanism 400 is further configured to prevent or oppose the jaws 108 moving apart, i.e. in a direction from the fully clamped configuration of the jaws 108 to the fully open configuration of the jaws 108.

In this embodiment, return mechanism 404 is coupled to the jaws 108 and acts on the jaws 108 to force the jaws 108 apart, i.e. to move the jaws 108 in a direction from the fully clamped configuration of the jaws 108 to the fully open configuration of the jaws 108. The return mechanism 404 may include a spring mechanism for moving the jaws 108.

In this embodiment, the force with which the ratchet mechanism 400 prevents or opposes the jaws 108 moving apart is much greater than the force with which the return mechanism 404 moves the jaws 108 apart. Thus, when the ratchet mechanism 400 is engaged with the jaws 108, the jaws 108 prevented from moving apart.

The release pin 406 is coupled to the ratchet mechanism 400. Pressing, or performing some other action, on the release pin 406 causes the release pin 406 to act of the ratchet mechanism 400, thereby disengaging the ratchet mechanism 400 from the jaws 108. The ratchet mechanism 400 being disengaged from the jaws 108 means that the ratchet mechanism 400 no longer prevents or opposed the jaws 108 from moving apart, and thus, in this embodiment, the jaws 108 are moved apart by the return mechanism 404.

In this way, i.e. by operating the release pin 406, the user may operate the gripping device 100 to release a gripped object.

Figures 5 and 6 are a schematic illustration (not to scale) showing further details of certain parts of the first body portion 104. Figures 5 and 6 show a side view cross section of part of the first body portion 104.

In this embodiment, the first elongate member 110 comprise hole (hereinafter "first hole" 500) along its length. Also, the second elongate member 112 comprises a series of spaced apart through holes (hereinafter "second holes" 502) along its length.

The handle 116 comprises a protrusion 504 at its upper end. The protrusion 504 is sized to fit through the first hole 500 and the second holes 502.

The handle 116 is coupled at its lower end to the first arm mount 114 via a spring 506 so that the handle may be moved up and down along its length with respect to the other components of the first body portion 104, e.g. with respect to the elongate members 110, 112 and the first arm mount 114.

In particular, in this embodiment, the handle 116 may be moved (for example, by the user gripping the handle and moving his or her wrist) up or down along its length between a first position (shown in Figure 5), and a second position (shown in Figure 6).

In its first position (Figure 5), the protrusion 504 of the handle 116 is positioned through aligned first and second holes 500, 502, i.e. through the elongate members 110, 112. This prevents or opposes the second elongate member 112 moving relative to the first elongate member 110, and the relative positions of the elongate members 100, 112 are fixed. In this first position, the spring 506 may force the handle against the elongate members 110, 112.

In its second position (Figure 6), the protrusion 504 of the handle 116 does not pass through at least a second hole 502, and preferably also the first hole 500. In other words, the handle 116 is moved downwards such that a tip of the protrusion 504 is below a lower surface of the second elongate member 112. In this position, the handle 116 no longer prevents or opposes the second elongate member 112 moving relative to the first elongate member 110. Thus, the second elongate member 112 may slide along the length of the first elongate member 110 so as to extend or reduce the length of the gripping device 100 (i.e. to increase or decrease a distance between the jaw portion 102 and the arm 200 of the user). The new relative positions of the first and second elongate members 100, 112 may be fixed by the handle 116 being returned to its first position.

To move the handle 116 from its first position to its second position, the user may move his or her arm to overcome the force exerted on the handle 116 by the spring 506. The user may allow the handle 116 to return towards its first position from its second position by releasing the downwards force on the handle 116 and allowing the spring 506 to force the handle upwards.

Thus, the gripping device 100 is advantageously extendible to allow a user to grip objects that are further away. In some embodiments, the slidable mounting of the elongate members 110, 112 allows for the length of the first body portion 104 to be increased by up to 75%

Advantageously, the arm mounts tend to uniformly distributes the load across the arm and reduce strain on the user's wrist.

The gripping device tends to reduce a need for hand motor movements of a user. The gripping device tends allow greater loads to be lifted. The gripping device tends provide improved control compared to conventional devices. The gripping device tends to help prevent repetitive strain injury to a user.

The inner faces of one or more of the arm mounts may be lined with an easy clean polyethylene foam to provide comfort and improved hygiene.

An inside surface of the chain systems may be coated with a flexible foam material to prevent injury to the user during operation.

The handle may be ergonomically designed for the user to hold. This tends to provide support and can help to increase control when using the gripping device. The handle may be coated in a thin rubber material to increase friction and provide comfort for the user.

Advantageously, the jaws may be replaceable. The jaws may have standard fitting structures facilitating replacement. Thus, the gripping device can be reconfigured using different jaws for different tasks.

Advantageously, the gripping device tends to be easy to manufacture using, for example, Additive Manufacturing techniques.

A specific embodiment of a gripping device and its method of use has been described for the purposes of illustrating the manner in which the invention is made and used. It should be understood that the implementation of other variations and modifications of the invention and its various aspects will be apparent to one skilled in the art, and that the invention is not limited by the specific embodiment described. Therefore, it is contemplated to cover the present invention and any and all modifications, variations, or equivalents that fall within the scope of the basic underlying principles disclosed and claimed herein.

## Claims

1. A gripping device comprising:
an elongate first body portion (104) having a first end and a second end opposite to the first end, the first body portion (104) comprising a first arm mount (114) for fitting to a forearm of a user;
a jaw portion (102) comprising a pair of jaws (108) moveable relative to each other between a fully open position and a fully clamped position thereof, the jaw portion (102) being coupled to the first end of the first body portion (104);
a second body portion (106) coupled to the second end of the first body portion (104), and moveable with respect to the first body portion (104), the second body portion (106) comprising a second arm mount (124) for fitting to an upper arm of a user; and
a pull member (126) operatively coupled between the jaw portion (102) and the second body portion (106), the pull member (126) being at least partially housed within the first body portion (104); wherein
movement of the second body portion (106) with respect to the first body portion (104) is operative to move the pull member (126) thereby to cause the pull member (126) to act on the jaw portion (102) so as to cause the jaws (108) to move from at or proximate to the fully open position thereof towards the fully clamped position thereof.

2. A gripping device (100) according to claim 1, wherein the jaw portion (102) further comprises a ratchet system (400) configured to oppose movement of the jaws (108) from at or proximate to the fully clamped position thereof towards the fully open position thereof.

3. A gripping device (100) according to claim 2, wherein the jaw portion (102) further comprises a release mechanism (404) for releasing the ratchet system (400) so as to allow movement of the jaws (108) from at or proximate to the fully clamped position thereof towards the fully open position thereof.

4. A gripping device (100) according to claim 3, wherein the jaw portion (102) further comprises a first spring mechanism for moving the jaws (108) from at or proximate to the fully clamped position thereof towards the fully open position thereof responsive to the release mechanism (404) releasing the ratchet system (400).

5. A gripping device (100) according to any of claims 1 to 4, wherein the first body portion (104) is selectively extendible such that a distance between the jaw portion (102) and the second body portion (106) can be varied.

6. A gripping device (100) according to any of claims 1 to 5, wherein:
the first body portion (104) further comprises:
a first elongate member (110); and
a second elongate member (112) slidably mounted to the first elongate member (110);
the jaw portion (102) is coupled to the second elongate member (112); and
the second body portion (106) is coupled to the first elongate member (110).

7. A gripping device (100) according to claim 6, wherein:
the first body portion (104) further comprises a handle portion (116) moveable between a first position and a second position thereof;
the first position of the handle portion (116) is such that the handle portion (116) is engaged with the first elongate member (110) and the second elongate member (112) thereby to fix the relative positions of the first elongate member (110) and the second elongate member (112); and
the second position of the handle portion (116) is such that the handle portion (116) is not engaged with at least one of the first elongate member (110) and the second elongate member (112) thereby allowing the relative movement of the first elongate member (110) and the second elongate member (112).

8. A gripping device (100) according to claim 7, wherein the first body portion (104) further comprises a second spring mechanism (506) for moving the handle portion (116) from the second position thereof to the first position thereof.

9. A gripping device (100) according to any of claims 1 to 8, wherein, when the first arm mount (114) is fitted to the forearm of a user and the second arm mount (124) is fitted the upper arm of that user, the movement of the second body portion (106) with respect to the first body portion (104) that moves the pull member (126) and thereby causes the pull member (126) to act on the jaw portion (102) so as to cause the jaws (108) to move from at or proximate to the fully open position thereof towards the fully clamped position thereof corresponds to the user's arm being straightened.

10. A gripping device (100) according to any of claims 1 to 9, wherein:
further movement of the second body portion (106) with respect to the first body portion (104) is operative to move the pull member (126) and thereby causes the pull member (126) to act on the jaw portion (102) so as to cause the jaws (108) to move from at or proximate to the fully clamped position thereof towards the fully open position thereof; and
the further movement is in an opposite direction to that of the movement of the second body portion (106) with respect to the first body portion (104) that moves the pull member (126) and thereby causes the pull member (126) to act on the jaw portion (102) so as to cause the jaws (108) to move from at or proximate to the fully open position thereof towards the fully clamped position thereof.

11. A gripping device (100) according to claim 10, wherein, when the first arm mount (114) is fitted to the forearm of a user and the second arm mount (124) is fitted the upper arm of that user, the further movement of the second body portion (106) with respect to the first body portion (104) corresponds to the user's arm being bent at an elbow of the user.

12. A gripping device (100) according to any of claims 1 to 11, wherein the pull member (126) is a cable.

13. A gripping device (100) according to any of claims 1 to 12, wherein at least part of the pull member (126) is housed in a sleeve.

14. A gripping device (100) according to any of claims 1 to 13, wherein the second body portion (106) comprises a roller chain system (122) that, when the first arm mount (114) is fitted to the forearm of a user and the second arm mount (124) is fitted the upper arm of that user, is located at an elbow of that user.

15. A method of providing a gripping device (100), the method comprising:
providing an elongate first body portion (104) having a first end and a second end opposite to the first end, the first body portion (104) comprising a first arm mount (114) for fitting to a forearm of a user;
coupling a jaw portion (102) the first end of the first body portion (104), the jaw portion (102) comprising a pair of jaws (108) moveable relative to each other between a fully open position and a fully clamped position thereof;
coupling a second body portion (106) to the second end of the first body portion (104) such that the second body portion (106) is moveable with respect to the first body portion (104), the second body portion (106) comprising a second arm mount (124) for fitting to an upper arm of a user; and
coupling a pull member (126) between the jaw portion (102) and the second body portion (106) such that the pull member (126) is at least partially housed within the first body portion (104) and such that movement of the second body portion (106) with respect to the first body portion (104) is operative to move the pull member (126) thereby to cause the pull member (126) to act on the jaw portion (102) so as to cause the jaws (108) to move from at or proximate to the fully open position thereof towards the fully clamped position thereof.
